# DEMANDE DE BREVET EUROPEEN

(11) **EP 1 602 347 A1**
(43) Date de publication de la demande: **07.12.2005**
(21) Numéro de dépôt: 04102543.8
(22) Date de dépôt: 04.06.2004
(51) Int. Cl.: A61F 5/56

(54) **Orthèse d'avancement mandibulaire**

(71) Demandeur: Magnin, Georges, 2314 La Sagne (CH)
(72) Inventeur: Magnin, Georges, 2314 La Sagne (CH)
(74) Mandataire: P&TS Patents & Technology Surveys SA

(57) **Abrégé**

Orthèse d'avancement mandibulaire pour combattre le ronflement et les apnées du sommeil. L'orthèse se compose de deux coques (1, 2) thermoformables destinées à envelopper l'arcade supérieure et inférieure. Afin de pouvoir s'adapter aux variations individuelles de conformation, l'orthèse comprend une armature articulée comportant des éléments rigides et souples, immergée dans la matière souple thermoformable ou surmoulée autour de celle-ci. Les articulations (12, 13, 23) de l'armature permettent à l'orthèse de suivre la courbe de l'arcade dentaire et de s'adapter aux irrégularités de position des dents. L'orthèse de l'invention est équivalente en confort et efficacité à une orthèse personnalisée réalisée par un orthodontiste ou un technicien dentaire, mais son coût est de 6 à 10 fois inférieur.

## Description

### Domaine technique

La présente invention se rapporte au domaine des orthèses buccales, et notamment à une orthèse pour le traitement du ronflement et de l'apnée nocturne.

### Technique antÃ©rieure

Le ronflement est une manifestation sonore bien connue dont les causes anatomiques et physiologiques sont liées à une hypotonie musculaire qui apparaît lors de l'endormissement. En effet la mandibule, normalement maintenue par l'action des muscles, tend à reculer pendant le sommeil, entraînant dans son mouvement la langue vers l'arrière de la bouche et provoquant ainsi une diminution du calibre des voies aériennes supérieures. Les turbulences du flux respiratoire qui en résultent, amplifiées par des phénomènes de vibration et de résonance du voile du palais et des parties molles adjacentes sont à l'origine du bruit du ronflement qui peut, dans certains cas, atteindre une intensité extrêmement élevée.

Le ronflement ne se limite pas à une gêne, même sévère, de la qualité du sommeil des familiers et des proches de la personne atteinte. Parfois, et notamment chez les individus présentant une macroglossie ou une cavité buccale de petites dimensions, ou si la laxité des tissus est élevée, l'obstruction du canal de respiration peut être grave ou complète, entraînant de fait des interruptions de la respiration, ou apnées obstructives du sommeil.

Dans ce cas, le ronflement est un aspect d'une véritable pathologie qui se manifeste également par la fragmentation du sommeil entraînant une somnolence diurne et une baisse des capacités de concentration. L'apnée entraîne une hypoxie, une bradycardie suivie d'une tachycardie avec risques hypertensifs. Cette apnée est susceptible de déclencher un arrêt cardio-respiratoire nocturne.

On sait traiter efficacement le ronflement et l'apnée nocturne par port nocturne d'un masque nasal insufflant de l'air sous pression dans le pharynx. Cette solution est néanmoins excessivement contraignante pour le porteur et nécessite des calibrations individuelles pour chaque patient, ainsi que des contrôles d'efficacité polysomnographiques. Pour ces raisons, l'application de ce type de traitement est limitée aux syndromes obstructifs et ne se justifie pas dans le traitement du simple ronflement. A la longue, le traitement est susceptible d'entraîner des manifestations nasales du type de rhinites ou d'escarres cutanées rendant impossible le port du masque.

On sait aussi traiter le ronflement et les apnées du sommeil par une opération de résection du voile du palais, éventuellement complétée par une chirurgie d'avancement maxillaire. Cette solution est toutefois invasive et douloureuse, et l'amélioration qui en dérive est seulement transitoire. De plus, elle peut impliquer des changements de la morphologie buccale pouvant perturber la déglutition. Cette opération très invasive est désormais pratiquement abandonnée.

On a également proposé pour combattre le ronflement, des appareils intra-buccals s'accrochant aux arcades dentaires supérieure et inférieure, permettant de propulser antérieurement la mandibule, même en l'absence d'une tension musculaire suffisante. La demande de brevet **DE 198628** décrit un exemple de dispositif monobloc de ce type de type immobilisant complètement la mandibule en position avancée. L'immobilisation de la mandibule est gênante pour le porteur, notamment en ce qu'elle interfère avec la déglutition et peut provoquer des syndromes algo-disfonctionnels de l'articulation temporo-mandibulaire.

Le brevet **EP 0845962 B** décrit un dispositif articulé thermoformable combinant des élément souples et des éléments semi-rigides, et autorisant une certaine liberté de mouvement de la mandibule tout en la propulsant en avant. Ce dispositif se compose de deux coques réalisées en un matériau thermoformable. De cette manière, chaque patient peut adapter les coques à sa propre conformation en les chauffant dans un bain d'eau porté à une température suffisante à ramollir la couche thermoformable, puis en les moulant sur l'arcade correspondante.

La conformation des dents et des arcades dentaires varie toutefois énormément d'un individu à l'autre, soit parce que les arcades peuvent être plus ou moins larges chez chaque individu, soit parce que les dents ne sont pas régulièrement positionnées le long de chaque arcade. Les dispositifs thermoformables connus ne présentent qu'une adaptabilité très limitée et ne peuvent pas s'adapter à tout le spectre de conformations possibles.

Lorsque l'orthèse ne peut pas s'adapter parfaitement à la conformation dentaire individuelle, on constate une réduction d'efficacité, une instabilité du dispositif, des tensions excessives sur la dentition, ainsi qu'une adaptation du système très inconfortable. En plus, suite aux mouvements involontaires de serrage de la mandibule, des efforts mécaniques élevés et concentrés peuvent se produire sur les éléments semi-rigides des coques, pouvant conduire à la rupture du dispositif.

Une autre limitation de ce type de dispositifs est que, si l'orthèse ne s'adapte pas parfaitement à la conformation dentaire, elle peut, pendant l'usage, exercer des forces latérales sur les dents, même de grande intensité. Ces forces latérales peuvent, avec le temps, conduire à un déplacement et à une déstabilisation des dents, et à leur perte définitive.

II est aussi connu de réaliser des orthèses d'avancement mandibulaire spécialement fabriquées pour s'adapter à la conformation dentaire du porteur. Dans ce cas, chaque orthèse est fabriquée comme une pièce unique d'après les empreintes du maxillaire et de la mandibule du porteur, par des procédés conventionnels dans le domaine de l'orthodontie. On conçoit facilement que le coût de fabrication de ce type d'appareil personnalisé est nettement plus élevé que celui d'une orthèse adaptable fabriquée en série.

Une autre limitation des dispositifs mandibulaires connus, tel que par exemple les dispositifs décrits par **EP 0845962 B**, qui autorisent une certaine liberté de mouvement de la mandibule est que cette dernière peut, à la suite de mouvements involontaires dans le sommeil, abandonner la position propulsée en avant. Le dispositif perd alors entièrement son efficacité. Dans ces cas la liberté de rétropulsion va paradoxalement à l'encontre du but recherché, de l'avancement mandibulaire.

La position du porteur pendant le sommeil peut aussi influencer négativement le bon fonctionnement des dispositifs mandibulaires connus. Il est par exemple fréquent que la mandibule abandonne la position propulsée en avant lorsque la tête est positionnée sur le côté.

II est aussi connu que certaines phases du sommeil sont caractérisées par une activité et une tonicité musculaire très importantes. Une fois ces épisodes de sommeil agité terminés, les dispositifs de l'art antérieur ne sont pas toujours en mesure de reporter automatiquement la mandibule dans la position thérapeutique propulsée.

Un but de la présente invention est de proposer une orthèse d'avancement mandibulaire exempte des inconvénients de l'art antérieur.

Un autre but de la présente invention est de proposer un dispositif intra-buccal contre le ronflement pouvant s'adapter à une très grande partie de conformations individuelles sans risques irréversibles pour la dentition de l'utilisateur.

Un autre but de la présente invention est de proposer un dispositif intra-buccal contre le ronflement plus confortable que les dispositifs connus.

Encore un but de la présente invention est de proposer une orthèse d'avancement mandibulaire adaptable à la conformation dentaire du porteur présentant un confort et une efficacité équivalents à ceux d'une orthèse personnalisée et un coût de fabrication réduit.

Ces buts sont atteints par les produits et le procédé qui sont l'objet des revendications indépendantes des catégories correspondantes et notamment par une orthèse d'avancement mandibulaire comprenant deux coques, aptes à envelopper la première l'arcade dentaire supérieure et la seconde l'arcade dentaire inférieure de la cavité buccale, comprenant des moyens de propulsion reliant lesdites première et seconde coque, propres à exercer une force de propulsion sur ladite seconde coque dans le sens postéro-antérieur, caractérisé en ce que au moins une desdites première et seconde coque comprend une armature articulée autorisant une déformation pour s'adapter à la conformation dentaire du porteur.

### Description des dessins

L'invention sera mieux comprise à la lumière de la description, des revendications et des figures suivantes dans lesquelles notamment :

Les figures 1 et 2 représentent schématiquement une orthèse d'avancement mandibulaire de type connu ;

La figure 3 représente schématiquement un dispositif comprenant des stries de glissement convexes et permettant un passage d'air selon un aspect de la présente invention.

La figure 4 représente schématiquement un dispositif comprenant une armature flexible externe selon un aspect de la présente invention.

La figure 5 représente schématiquement une variante ultérieure d'un dispositif comprenant une armature flexible externe selon un aspect de la présente invention.

La figure 6 représente schématiquement un dispositif comprenant une armature flexible interne selon un aspect de la présente invention.

Les figures 7 et 8 représentent schématiquement deux sections du dispositif de la figure 5.

La figure 9 représente schématiquement une section du dispositif de la figure 5 inséré dans une gouttière de prise d'empreinte.

La figure 10 représente schématiquement un indicateur de température étalonné utilisé dans le procédé de prise d'empreinte selon l'invention.

Les figures 11 et 12 représentent schématiquement un autre mode de réalisation de l'invention comprenant une surface inclinée par rapport au plan de glissement principal.

Les figures 13, 14 et 15 représentent schématiquement un autre mode de réalisation de l'invention.

Les figures 16 et 17 montrent schématiquement un autre mode de réalisation de l'invention comprenant une languette élastique.

La figure 18 représente schématiquement une coupe de l'extrados d'une orthèse selon l'invention selon le plan AA de la figure 12.

### Mode pour l'invention

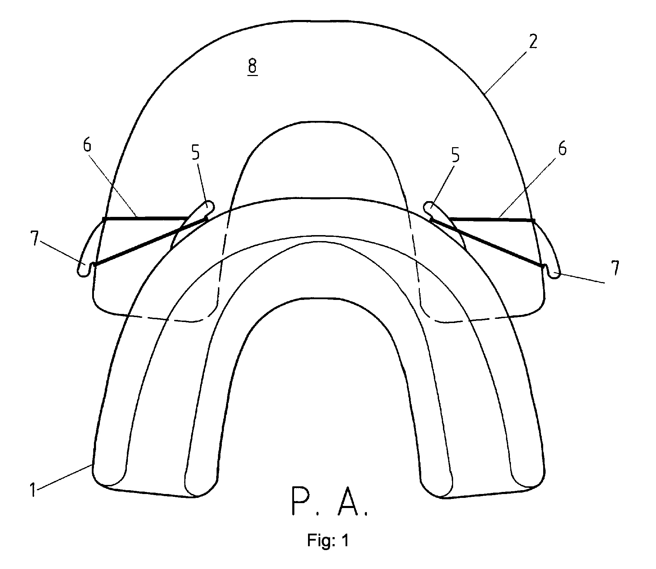

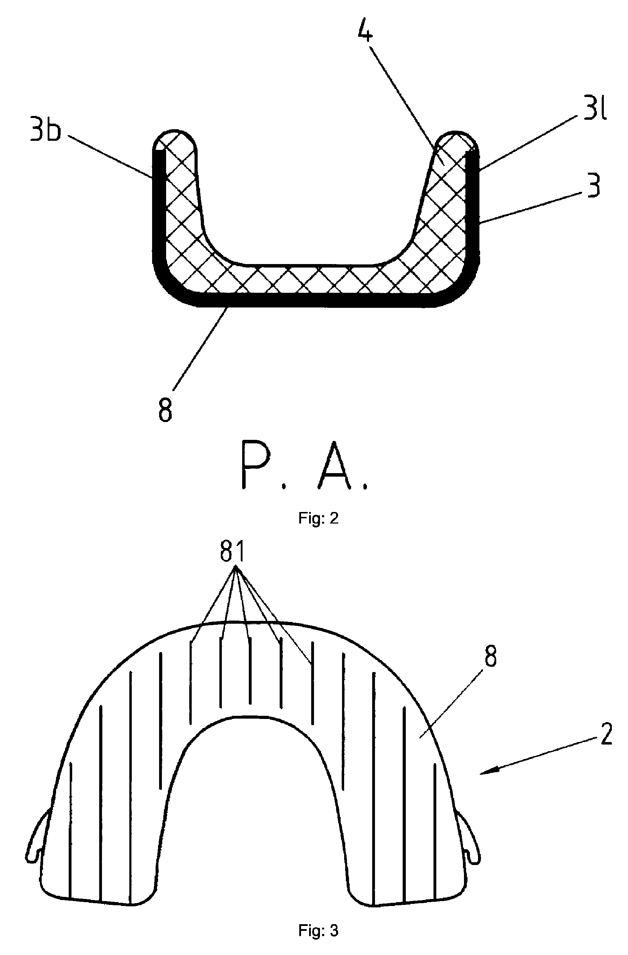

La figure 1 représente un dispositif intra-buccal contre le ronflement de type connu. Il se compose de deux coques 1, 2, destinées à envelopper l'arcade dentaire supérieure, respectivement inférieure. Avec référence à la figure 2, les coques 1 et 2 présentent un extrados essentiellement rigide 3 sur lequel un intrados thermoformable 4 est surmoulé. Les coques sont reliées entre elles par les crochets 5 et 7 et les boucles élastiques 6, pour exercer une force de propulsion sur la mandibule, tout en permettant une certaine liberté de mouvement. L'extrados rigide de ce dispositif est incapable de suivre une dentition irrégulière.

Lors de l'usage, les surfaces planes 8 des deux extrados se trouvent en contact le long du plan d'occlusion. Les mouvements de latéralité et de propulsion/rétropulsion de la mandibule comportent donc un glissement de ces surfaces 8 l'une sur l'autre.

Pour permettre une adaptation personnalisée de cet appareillage chaque patient peut adapter les coques à sa propre conformation en moulant l'intrados thermoformable sur l'arcade correspondante, après l'avoir ramolli par immersion dans un bain d'eau à une température appropriée.

Le dispositif de la figure 1 présente donc une bonne conformabilité pour autant que toutes les dents se positionnent en correspondance de l'intrados souple. Il est toutefois fréquent que les dents ne suivent pas exactement la courbure des coques 1 et 2, et qu'une dent se trouve en correspondance de l'extrados 3 lors de la prise de l'empreinte. En cette situation l'orthèse ne peut pas du tout s'adapter à l'arcade.

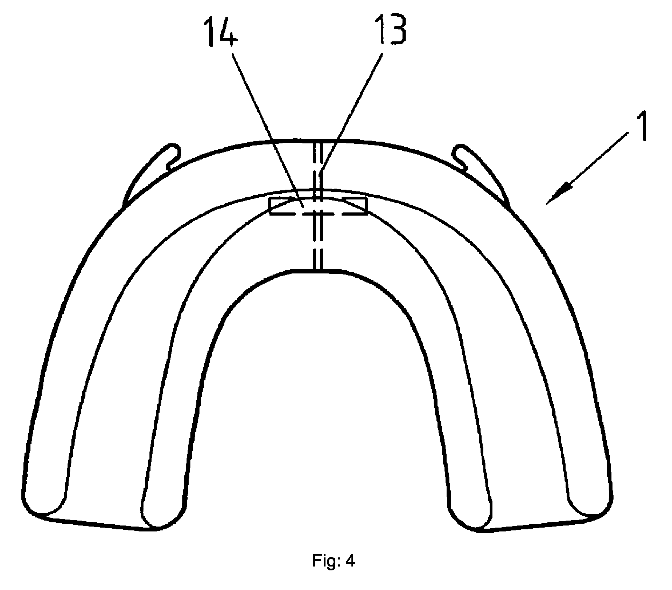

La figure 4 représente une coque d'un dispositif comprenant une armature articulée selon l'invention. Dans ce dispositif, l'extrados comprend au moins un joint flexible de manière à réaliser une armature articulée pour suivre la conformation dentaire du patient, même dans le cas d'une implantation irrégulière des dents.

Dans cette forme d'exécution simple, le dispositif de l'invention se limite au seul point central de flexion, situé en correspondance du plan sagittal. Le joint flexible est obtenu en pratiquant une fente 13 dans l'extrados 3. La flexibilité relativement plus élevée en correspondance de la fente 13 permet l'articulation de l'extrados 3. Un élément flexible métallique ou plastique 14 peut être inséré pour relier les deux moitiés droite et gauche de l'extrados 3. Le système de l'invention peut comprendre une ou plusieurs fentes selon le degré d'adaptabilité recherché.

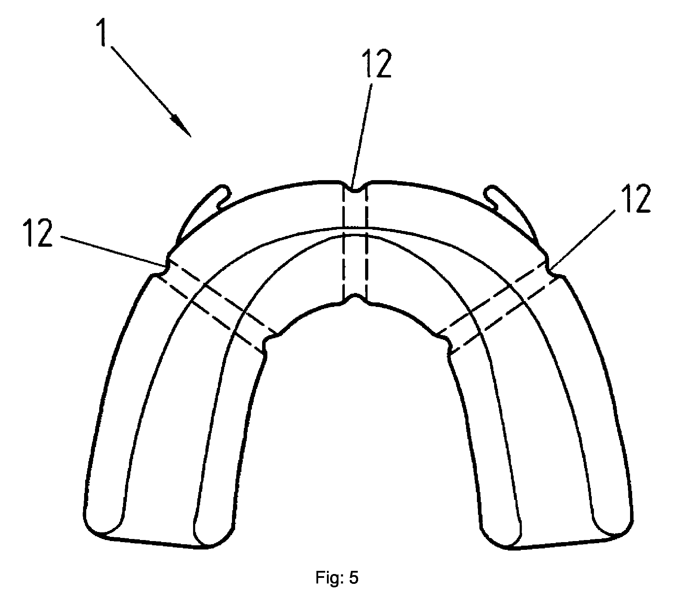

La figure 5 représente, par souci de simplification, la coque supérieure 1 uniquement. Bien entendu, les caractéristiques propres à ce mode de réalisation de l'invention peuvent aussi être avantageusement incorporées dans la coque inférieure 2.

La fente centrale 13 permet aussi l'alignement correct des deux coques lors de la prise d'empreinte. Optionnellement la même fonction peut être assuré par des marques, de préférence convexes, gravées à cet effet sur les deux coques.

Dans le cadre de la présente invention, les boucles élastiques pourraient être remplacée par d'autres moyens équivalents, par exemple par des ressorts, des pistons, par des languettes flexibles réalisés dans l'une ou dans l'autre coque ou par des aimants.

Avantageusement, l'armature comporte une pluralité de joints flexibles, pour s'adapter à un plus grand spectre de conformations dentaires. La figure 5 représente une coque d'un dispositif selon l'invention muni d'un extrados formant une armature articulée. L'extrados 3 comporte dans ce cas trois joints flexibles 12 réalisés par un amincissement des parois externes. De préférence, l'extrados 3 sera réalisé en un polymère susceptible d'être formé par moulage, comportant une bonne élasticité et pouvant tolérer un grand nombre de cycles de flexion, par exemple en un élastomère thermoplastique.

Les matériaux composants l'extrados et l'intrados sont sélectionnés afin de procurer une bonne adhésion entre ces deux parties. Une bonne compatibilité chimique est donc importante pour parvenir à ce résultat. L'adhésion entre extrados et intrados peut être encore améliorée en prévoyant des nervures ou des cavités dans l'extrados pour offrir une surface d'accrochage lors du surmoulage.

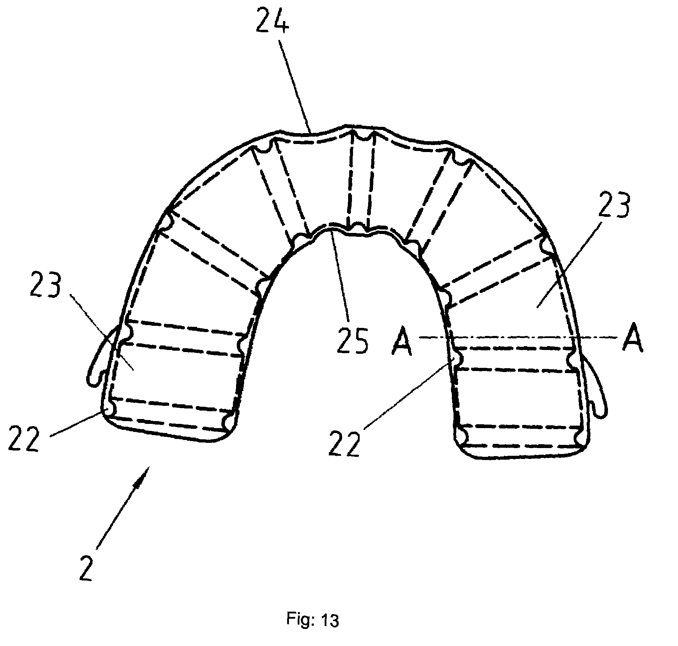

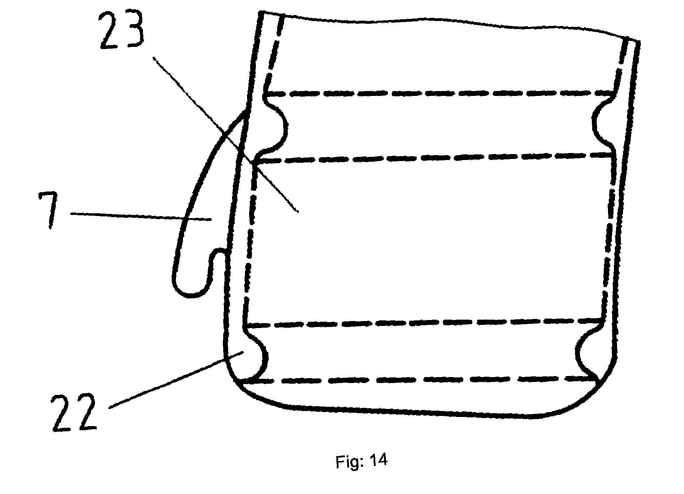

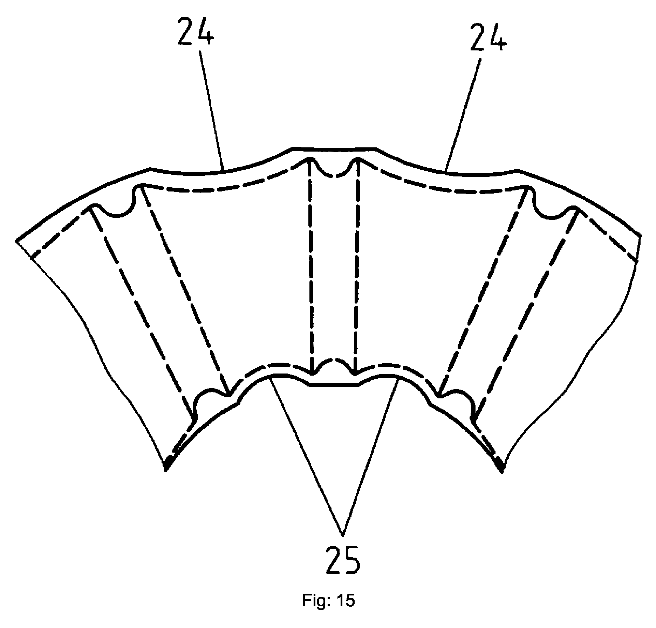

Les figures 13, 14 et 15 montrent l'extrados flexible de la coque inférieure 8 d'une orthèse selon une autre variante de l'invention.

Dans ce mode de réalisation les joints flexibles sont réalisés par amincissement des parois intérieures de l'extrados, ce qui présente l'avantage de conserver une surface extérieure parfaitement lisse, et de ne pas laisser des surfaces extérieures concaves, difficiles à nettoyer. L'extrados comporte selon cette variante une alternance de sections minces 23 et de nervures plus rigides 22. Les sections minces 23 travaillent essentiellement en flexion et en extension, et permettent à l'extrados de suivre la forme générale de l'arcade dentaire. Les nervures 22, en revanche, présentent une rigidité suffisante pour garantir la stabilité latérale de l'orthèse contre les dents, et notamment contre les faces linguales et labiales des dents.

II est important, pour la stabilité de l'orthèse et pour la santé dentaire, que les flancs 3l et 3b de l'extrados exercent, par leur rigidité, deux force latérales opposées sur les faces linguales et labiales des dents, respectivement. L'orthèse de l'invention, par sa conformabilité, permet le positionnement symétrique des deux flancs 3l et 3b par rapport aux dents, et par conséquent une tenue efficace et sans efforts asymétriques, même en présence d'un positionnement irrégulier des dents.

Avantageusement les zones plus sollicitées en extension et en compression présentent des parois minces courbées 24, 25 pour pouvoir assurer des élongations et des compressions plus importantes, grâce à leur flexibilité. La courbure des parois 24 et 25 pourra être vers l'intérieur (concave) ou vers l'extérieur (convexe), selon la nécessité.

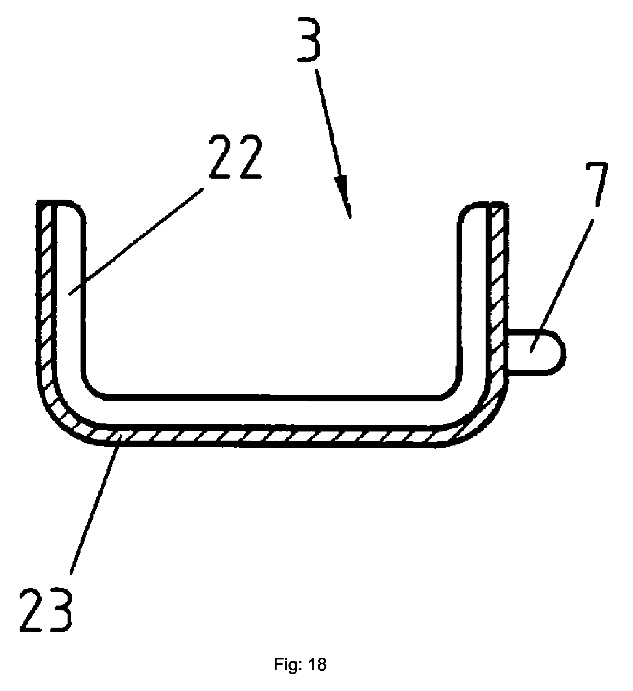

La figure 18 représente schématiquement une coupe de l'extrados 3 selon le plan AA de la figure 13. On peut voir la section mince 23, traversée par le plan de coupe AA, et la nervure 22 en arrière plan.

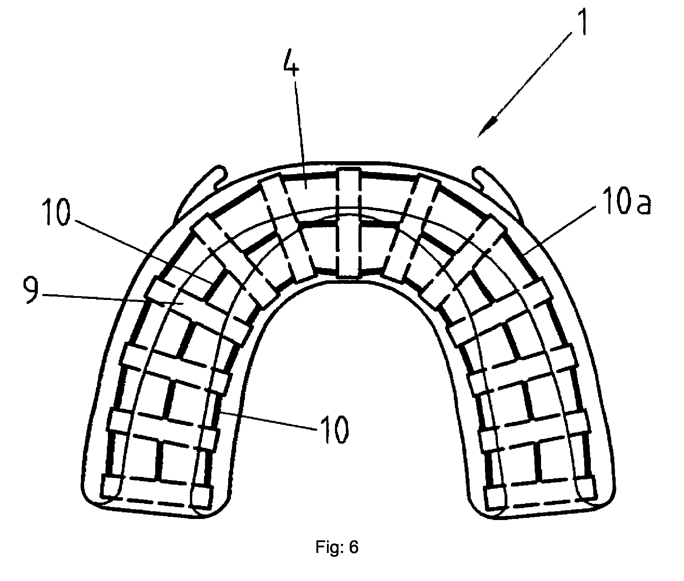

Un autre mode de réalisation de l'invention sera maintenant décrit avec référence à la figure 6. Dans ce mode de réalisation une armature flexible et articulée est insérée à l'intérieur d'une coque 4 en un matériau souple et thermoformable. L'armature flexible se compose d'une succession d'éléments 9 en forme de « U » rigides, ouverts vers l'arcade dentaire, reliés par des éléments flexibles 10 et 10a plus minces. Avantageusement l'armature articulée est réalisée en une seule pièce par moulage d'un matériau approprié comportant une bonne élasticité et déformabilité et pouvant tolérer un très grand nombre de cycles de flexion en section mince.

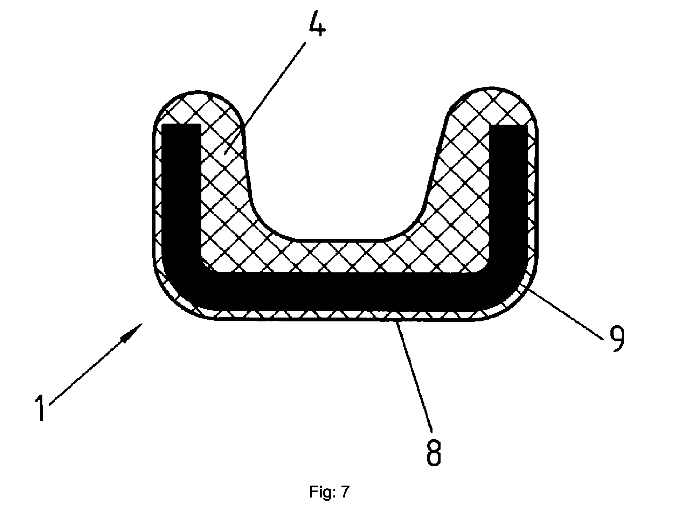

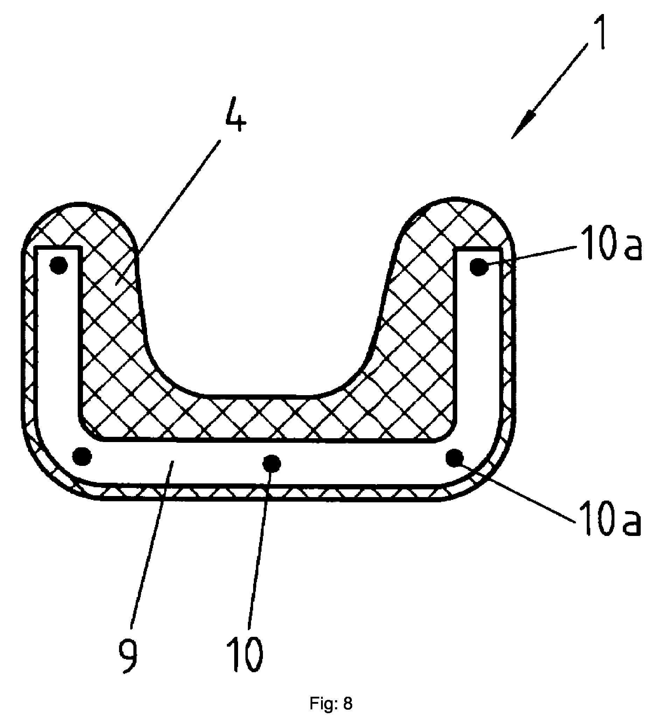

L'orthèse selon cette variante de l'invention présente une surface extérieure entièrement souple, et offre donc un confort supérieur aux dispositifs comportant des surfaces extérieures rigides ou semi-rigides. Les figures 7 et 8 montrent deux coupes de la coque 4 de la figure 6 en correspondance des éléments 9 et 10.

Un autre avantage de cette variante de l'invention est que les risques de décollement entre l'armature interne et la partie thermoformable sont inexistants.

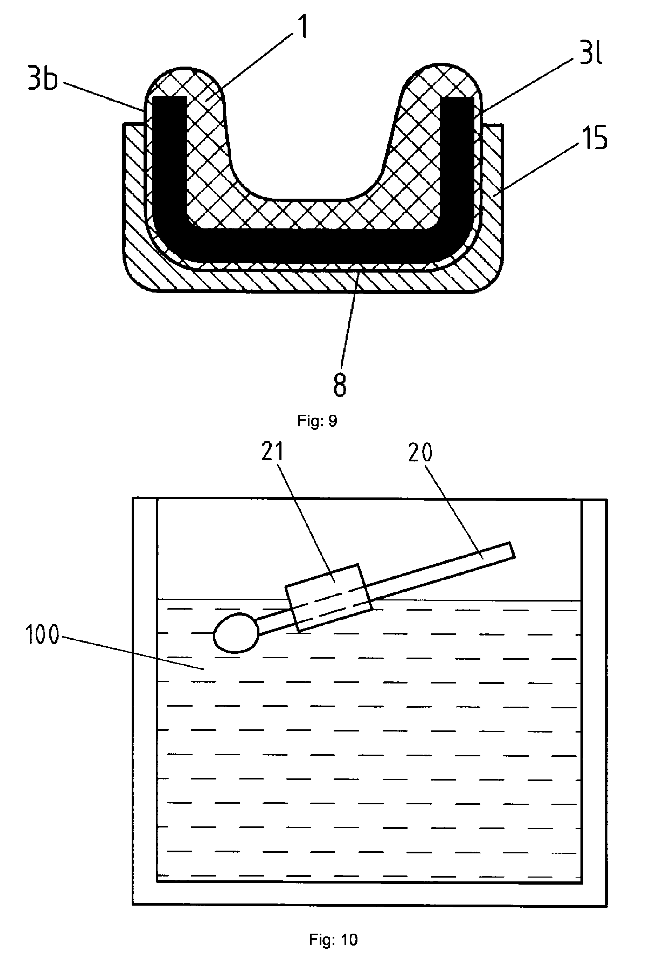

La figure 9 illustre un élément d'interface 15 qui intervient lors de la prise d'empreinte du dispositif de l'invention. Le but de ce dispositif est d'empêcher, pendant la prise d'empreinte, un écartement des deux flancs 3l et 3b, qui aurait l'effet de réduire la pression sur les faces linguales et labiales des dents et, en conséquence, la stabilité de l'orthèse. A cette fin, les coques sont insérées, pour la prise d'empreinte seulement, dans deux éléments d'interface en forme de gouttières souples jetables 15 qui empêchent la déformation de la surface 8 et l'écartement des flancs 31 et 3b.

II est également important que les deux coques qui composent l'orthèse présentent deux plans de glissement 8 parfaitement adaptés l'un à l'autre. L'usage des éléments d'interface 15 permet d'empêcher toute déformation desdits plans lors de la prise d'empreinte.

Une gouttière symétrique est prévue pour la coque inférieure. Une fois terminé le moulage des gouttières 1 et 2 sur les arcades, les éléments d'interface 15 peuvent être éliminés, après refroidissement des éléments thermoformables 4.

Ces éléments d'interface 15 peuvent aussi être avantageusement employés dans les autres modes de réalisation présentés, tout en restant dans le cadre de la présente invention.

Optionnellement, le plan de glissement 8 d'au moins une des coques 1 et 2 présente une pluralité de stries convexes parallèles 81, comme représenté sur la figure 3. Il a été constaté que les stries 81 agissent comme patins de glissement réduisant fortement le frottement entre les coques et facilitent le retour de la mandibule dans la position recherchée après un mouvement involontaire. En outre les stries 81 permettent de ménager un passage pour l'air entre les deux coques 1 et 2.

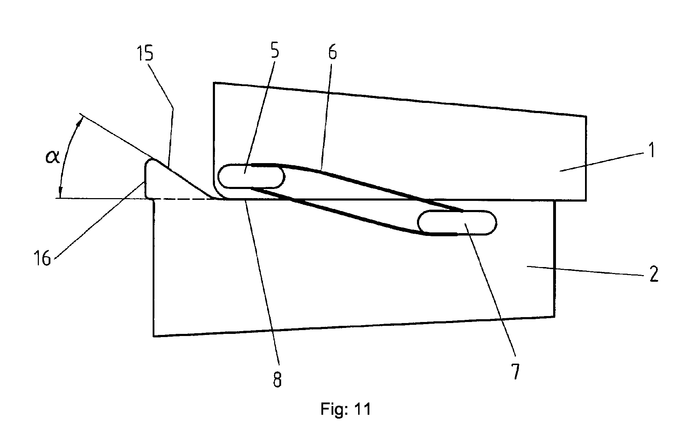

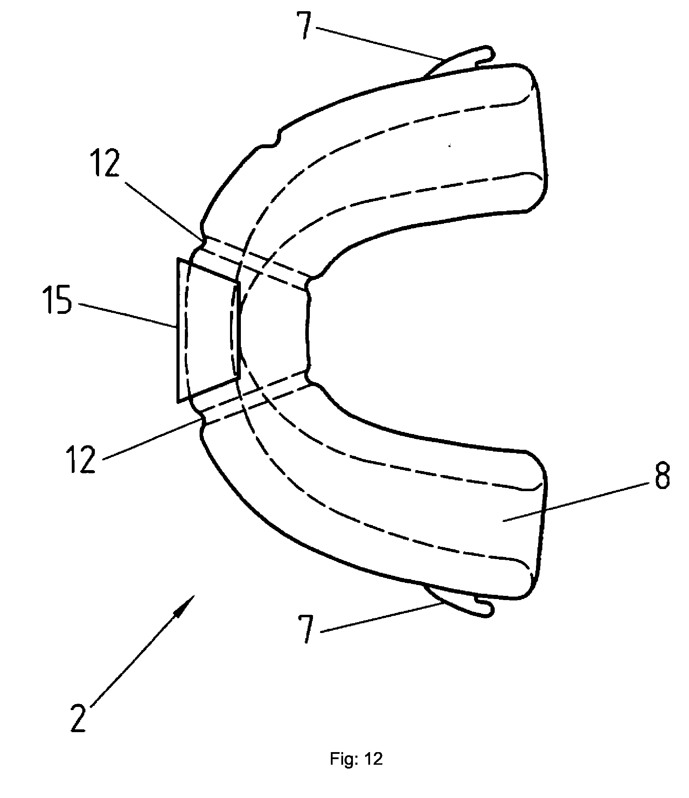

Une autre variante de l'invention sera maintenant décrite avec référence aux figures 11 et 12. Cette variante présente des caractéristiques tendant à assurer que la mandibule retourne à la position avancée après des mouvements involontaires de rétropulsion, durant certaines phases du sommeil, et en l'occurrence de certaines positions de la tête qui favorisent la rétropulsion, par exemple sur le côté.

On a vu que, pendant le sommeil, la mandibule se déplace parfois vers l'arrière de façon involontaire. Ces mouvements sont physiologiques pendant le sommeil et la déglutition et il ne serait pas envisageable de bloquer la mandibule dans une position propulsée statique.

La figure 11 représente les deux coques 1 et 2 d'une orthèse selon l'invention dans la position propulsée. La coque inférieure 2 présente, à l'extrémité antérieure une protubérance 16 ayant un plan incliné 15 juxtaposé à l'extrémité antérieure de la coque supérieure 1. Lors d'un mouvement de rétropulsion la coque 1 glisse sur le plan incliné 15 et de ce fait les coques 1 et 2 s'écartent légèrement.

La mandibule se trouve donc dans une situation instable car les boucles élastiques 6 tendent à faire glisser la coque supérieure 1 vers l'arrière. La surface inclinée 15 est orientée dans le sens d'augmenter la force de propulsion lors d'un mouvement de rétropulsion de la mandibule, aidant ainsi le maintien de la position propulsée. De cette manière, après des mouvements involontaires de rétropulsion, lorsque la tension musculaire cesse, la mandibule revient spontanément à la position de la figure 11. Il a été constaté qu'un angle a d'environ 40° permet de contrer efficacement les mouvements involontaires sans immobiliser la mandibule dans une position propulsée statique.

La figure 10 représente un thermomètre pour la prise de température d'un bain d'eau chaude destiné à faciliter la mise en forme des dispositifs de l'invention.

La prise d'empreinte comporte l'immersion préalable des coques dans un bain d'eau froide amené ensuite à une température prescrite destiné à ramollir la matière thermoformable 4. Selon les matériaux choisis la température de prise d'empreinte idéale se situe entre 70 et 90° C, typiquement 80 °C. Il a été constaté que les utilisateurs rencontrent des difficultés dans l'estimation de la température idéale, et que la prise d'empreinte est parfois imparfaite en raison d'une température trop faible ou trop élevée. Les conséquences de ces erreurs sont des empreintes trop légères ou bien des brûlures.

Les thermomètres traditionnels en verre on tendance à se déposer sur le fond du bain 100, et vont donc relever une température bien supérieure à la température prescrite du bain.

II existe aussi des indicateurs de température très légers, par exemple des thermomètres qui signalent la température par le changement de couleur d'un cristal liquide. Ces dispositifs sont facilement transportés par les mouvements de convection et le bouillonnement du liquide, de sorte que la lecture de ces dispositifs n'est pas facile.

Pour pallier à ces inconvénients et proposer un dispositif de lecture simple et économique, le thermomètre 20 comporte un flotteur asymétrique 21 de manière à déterminer une position d'équilibre hydrostatique inclinée par rapport à la verticale du thermomètre 20. Cette disposition permet une lecture aisée de la température.

De préférence la position d'équilibre du thermomètre est choisie de manière à ce que le bulbe sensible du thermomètre se trouve assez proche de la surface de l'eau, tout comme les coques flottantes 1 et 2, pour donner une lecture le plus proche possible de celle des coques elles-mêmes.

Pour simplifier encore la lecture le thermomètre 20 porte une seule marque graduée correspondante à la température prescrite.

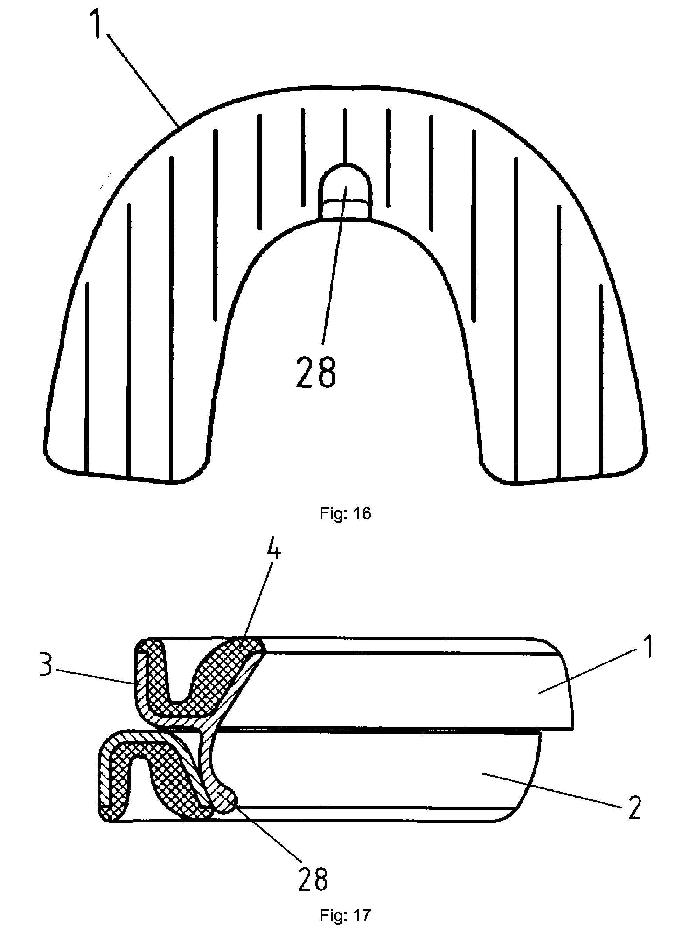

Avec références aux figures 16 et 17 un autre mode de réalisation de la présente invention prévoit une languette flexible 28 réalisée intégralement dans la coque supérieure 1 lors du moulage de l'extrados 3 de celle-ci. La languette 28 forme de préférence un monobloc avec la coque 1.

La languette 28, légèrement précontrainte s'appuie contre la coque inférieure 2 lorsque l'orthèse est dans sa position d'utilisation normale, et exerce une force de propulsion sur la coque inférieure dans le sens postéro-antérieur, positionnant ainsi la mandibule dans la position propulsée recherchée pour empêcher le ronflement. Par son élasticité et sa position centrale, la languette 28 autorise néanmoins une bonne liberté latérale de la mandibule lors des mouvements involontaires du sommeil, tout en repositionnant la mandibule en avant une fois le mouvement terminé.

La languette 28 peut aussi fonctionner en usage mixte, combiné avec des boucles élastiques et/ou des systèmes à ressort. Sa fonction de butée élastique est aussi très favorable durant les phases de sommeil marquées par une tonicité musculaire très active.

Dans le mode de réalisation représenté sur les figures, la languette élastique 28 est réalisée en monobloc avec l'extrados 3 de la coque supérieure 1 et se situe essentiellement sur le plan sagittal, à l'aplomb des dents incisives supérieures. D'autres dispositions, par exemple postérieurement, sont toutefois possibles et comprises dans la présente invention.

## Revendications

1. Orthèse d'avancement mandibulaire, comprenant une première coque (1) pour envelopper l'arcade dentaire supérieure et une seconde coque (2) pour envelopper l'arcade dentaire inférieure de la cavité buccale, l'orthèse comprenant des moyens de propulsion (6, 28) reliant lesdites première et seconde coque (1, 2), propres a exercer une force de propulsion sur ladite seconde coque dans le sens postéro-antérieur, **caractérisé en ce que** au moins une desdites première et seconde coques (1, 2) comprend une armature articulée, autorisant une déformation pour s'adapter à la conformation dentaire du porteur.

2. Orthèse selon la revendication 1, dans laquelle ladite armature articulée comporte des éléments relativement rigides, pour exercer une force de maintien contre les faces labiale et linguale des dents, et des éléments souples, pour permettre l'articulation et/ou la déformation de ladite armature.

3. Orthèse selon la revendication 1 ou 2, comprenant des éléments en matière plastique thermoformable (4) dans lesdites première et seconde coque (1, 2) pour s'adapter à la conformation dentaire du porteur.

4. Orthèse selon la revendication 1 ou 2dans laquelle lesdits moyens de propulsion comprennent des boucles élastiques (6).

5. Orthèse selon la revendication 1 ou 2dans laquelle lesdits moyens de propulsion comprennent au moins une languette élastique (28) dans l'une desdites première et seconde coque (1, 2).

6. Orthèse selon la revendication 3, dans laquelle ladite armature est à l'intérieur desdits éléments en matière plastique thermoformable (4).

7. Orthèse selon la revendication 3, dans laquelle ladite armature est à l'extérieur desdits éléments en matière plastique thermoformable.

8. Orthèse selon l'une des revendications de 1 à 7, dans laquelle chacune des dites première et seconde coque comprend un plan de glissement (8), lesdits plans de glissement desdites première et seconde coque étant en contact lors de l'utilisation normale de l'orthèse, comprenant des stries longitudinales convexes (81) sur au moins un desdits plans de glissement (8).

9. Orthèse selon l'une des revendications de 1 à 8 dans laquelle ladite première coque (1) et ladite seconde coque (2) comprennent des indices visuels pour faciliter l'alignement relatif desdites première et seconde coques.

10. Orthèse selon l'une des revendications de 1 à 9 dans laquelle au moins une desdites première et seconde coque comprend une surface inclinée (15) par rapport audit plan de glissement.

11. Orthèse selon la revendication 10, dans laquelle ladite surface inclinée est orientée dans le sens d'augmenter la force de propulsion lors d'un mouvement de rétropulsion de la mandibule.

12. Orthèse selon la revendication 10, dans laquelle ladite surface inclinée est disposée de manière à maintenir la mandibule en position propulsée.

13. Orthèse selon la revendication 10, dans laquelle ladite surface inclinée est disposée de manière à assurer que la mandibule retourne à la position propulsée après des mouvements involontaires de rétropulsion.

14. Procédé d'adaptation d'une orthèse d'avancement mandibulaire comprenant les étapes de :
- chauffer ladite orthèse par immersion dans un bain d'eau amené à une température prédéterminée ;
- insérer la première coque et la seconde coque de ladite orthèse dans un premier, respectivement second, élément d'interface amovible (15);
- positionner ladite orthèse dans la bouche du porteur ;
- exercer une pression pour mouler lesdits éléments thermoformables sur les arcades du porteur ;
- ôter lesdits premier et second élément d'interface amovible (15).

15. Procédé selon la revendication 12, **caractérisé en ce que** la température dudit bain d'eau (100) est déterminée par lecture d'un indicateur de température muni d'un flotteur (21), la position d'équilibre hydrostatique dudit indicateur de température étant inclinée par rapport à la verticale.

16. Procédé selon la revendication 13, **caractérisé en ce que** ledit indicateur de température comprend un thermomètre sans graduation (20), comprenant un indice visuel correspondant à ladite température prédéterminée.
